## Europäisches Patentamt

## European Patent Office

## Office européen des brevets

(11) Veröffentlichungsnummer: **0 397 983**
A2

# EUROPÄISCHE PATENTANMELDUNG

(21) Anmeldenummer: **90104886.8**

(22) Anmeldetag: **15.03.90**

(51) Int. Cl.5: **A61B 17/39**

(30) Priorität: **18.05.89 DE 3916161**

(43) Veröffentlichungstag der Anmeldung:
**22.11.90 Patentblatt 90/47**

(84) Benannte Vertragsstaaten:
**CH DE FR GB IT LI**

(71) Anmelder: **Richard Wolf GmbH**
**Pforzheimer Strasse 32**
**D-7134 Knittlingen(DE)**

(72) Erfinder: **Bitrolf, Ehrenfried**
**Tannenweg 8**
**D-7134 Knittlingen-Kleinvillars(DE)**

(74) Vertreter: **Wilcken, Thomas, Dipl.-Ing. et al**
**Musterbahn 1**
**D-2400 Lübeck(DE)**

(54) **Elektrochirurgisches Instrument.**

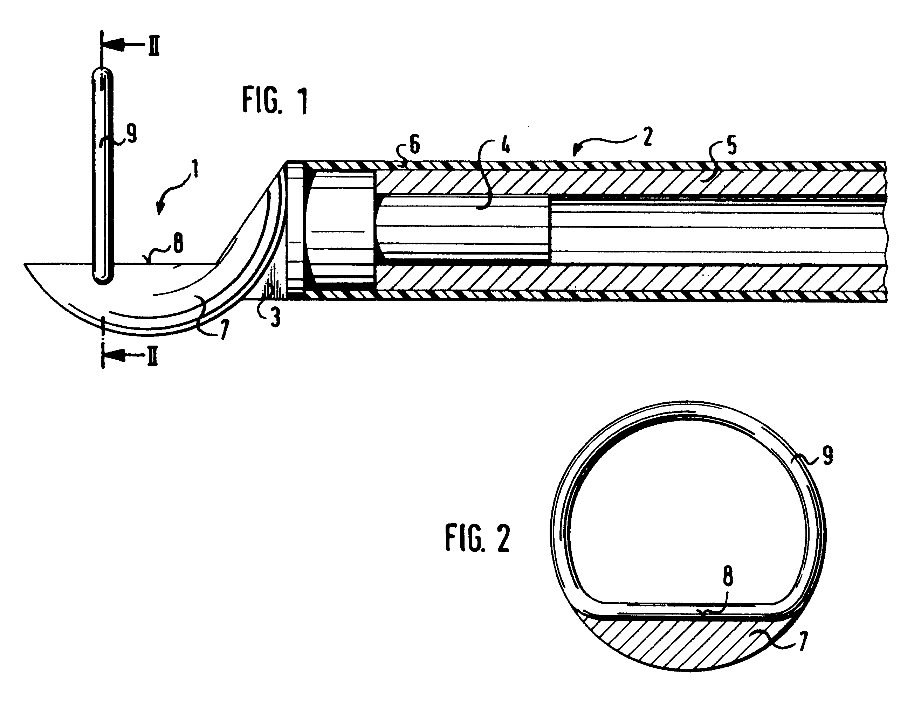

(57) Das vorgeschlagene elektrochirurgische Instrument (1) besitzt einen Sondenschaft (2), an dessen distalem Ende eine mit einer HF-Stromquelle verbindbare Elektrode (3) angeordnet ist. Um die Elektrode (3) sowohl zum Koagulieren als auch zum Resizieren unter Koagulation anwenden zu können, weist sie die Form eines Kugelabschnittes (7) auf, der mit einem Elektrodenring (9) bestückt ist. Dieser Elektrodenring ist im Winkel zu der ebenen Fläche (8) des Kugelabschnittes (7) aufragend angeordnet (Figur 1).

FIG. 1

EP 0 397 983 A2

## Elektrochirurgisches Instrument

Die Erfindung betrifft ein elektrochirurgisches Instrument mit einem Sondenschaft, an dessen distalem Ende ein mit einer HF-Stromquelle verbindbare Elektrode angeordnet ist.

Solche Instrumente sind in der medizinischen Technik allgemein bekannt. Sie werden bei der sogenannten Elektrokoagulation eingesetzt, einer Methode, bei welcher durch Wirkung des HF-Stromes in mit der Elektrode in Kontakt kommenden Gewebezellen aufgrund hoher Felddichte eine Überhitzung erzeugt wird, die zu einem Ausfallen kolloidaler Stoffe aus ihrer kolloidalen Lösung führt. Dabei sind die jeweili gen Instrumente bzw. deren unmittelbar mit dem biologischen Gewebe in Kontakt tretenden Elektroden an den jeweiligen Verwendungszweck bezüglich ihrer Raumform angepaßt ausgebildet. So sind beispielsweise aus der US 3 532 095 und dem DE-GM 84 23 501 elektrochirurgische Instrumente bekannt, die an ihrem distalen Ende jeweils eine kugelförmige Elektrode aufweisen, die leicht austauschbar in dem sich proximalwärts daran anschließenden Schaft angeordnet sind.

Aus der DE-AS 2 324 658 ist ferner eine HF-Sonde zum koagulieren von biologischem Körpergewebe bekannt, die zwecks Vermeidung einer großflächigen Massenelektrode als bipolare Elektrode ausgebildet ist, eine erste ringförmige und eine hierzu im Abstand angeordnete, halbkugelförmig ausgebildete und das distale Ende der HF-Sonde bildende zweite Elektrode aufweist.

Ein weiterer hier interessierender Stand der Technik ist aus der DE-OS 2 941 060 zu entnehmen, die eine Resektionsvorrichtung zeigt, bei der die von einem HF-Strom durchflossene Elektrode als Schlinge ausgebildet ist, die um einen aus der Körperoberfläche herausragenden Gewebeteil, beispielsweise Polypen oder dergleichen, legbar ist, so daß dieser Gewebeteil unter gleichzeitiger Koagulation der Trennstellen resizierbar ist. Die Resektionsschlinge ist zwecks Veränderbarkeit der Schlingenweite in einem rohrförmigen Schaft längsverschiebbar angeordnet.

Resektionsschlingen sind schließlich auch aus der DE-PS 2 525 982 bekannt. Sie werden für die Prostataresektion eingesetzt und ermöglichen ein streifenweises Abtragen, das heißt die Resektion von Gewebe unter gleichzeitiger Koagulation der Schnittflächen.

Die konstruktive Ausbildung der zur Gewebekoagulation bzw. Geweberesektion verwendeten Elektroden in Form von Sonden und Schlingen richtet sich im wesentlichen nach dem jeweiligen Verwendungszweck, so daß eine von diesem abweichende Anwendung, wenn überhaupt, nur zu einem geringen Teil möglich ist.

Besteht bei einem endoskopischen Eingriff beispielsweise im Rektum neben der Notwendigkeit einer Koagulation auch die einer Resektion, so sind nach dem bekannten Stand der Technik mindestens ein Instrument mit austauschbaren HF-Elektroden oder zwei separate Instrumente mit speziell ausgebildeten HF-Elektroden erforderlich.

Der Nachteil der bekannten Ausführungen besteht nun darin, daß einerseits die austauschbar am Instrument festlegbaren HF-Elektroden im Rektum verlorengehen können und andererseits eine zusätzliche Belastung des Patienten dadurch unvermeidlich ist, daß sowohl bei derartig ausgebildeten Instrumenten als auch bei Verwendung mehrerer speziell ausgebildeter Instrumente der endoskopische Eingriff aufgrund der für den Instrumentenwechsel erforderlichen Zeit verlängert wird.

Es ist daher die Aufgabe der Erfindung, diese den bekannten Ausführungen anhaftenden Nachteile zu vermeiden.

Die Lösung dieser Aufgabe geht von dem einleitend angeführten Instrument aus und kennzeichnet sich weiter dadurch, daß die Elektrode die Form eines Kugelabschnitts aufweist, der mit einem Elektrodenring bestückt ist, der von der ebenen Flä che des Kugelabschnittes aufragend angeordnet ist. Dabei kann der Elektrodenring zweckmäßigerweise so ausgebildet sein, daß sich die Ringebene desselben im wesentlichen senkrecht zu der Längsachse des Sondenschaftes erstreckt.

Die Vorteile einer derartig ausgebildeten Elektrode bestehen in der kombinierten Anwendbarkeit derselben sowohl zum Koagulieren als auch zum Resizieren unter Koagulation der Schnittflächen.

Nach einer weiteren Ausgestaltung der Erfindung kann der Elektrodenring derart geformt sein, daß die in der Ringebene desselben und durch den Kugelabschnitt verlaufend gedachte Querschnittsfläche eine kreisförmige Kontur aufweist, wobei die Ringebene zu der Längsachse des Sondenschaftes auch geneigt verlaufen kann. Bezüglich der Anwendungssicherheit erweist es sich als vorteilhaft, wenn der Elektrodenring mit dem Kugelabschnitt unlösbar verbunden ist.

Das erfindungsgemäße Instrument ist nachstehend anhand eines in der Zeichnung dargestellten Ausführungsbeispiels näher erläutert. Es zeigen:

Figur 1 eine Seitenansicht mit teilweisem Längsschnitt des Sondenschaftes,

Figur 2 eine Schnittdarstellung entlang der Linie II - II nach Figur 1.

Wie aus Figur 1 ersichtlich ist, besteht die Kombinationselektrode 1 aus einem Sondenschaft 2, in dessen distales Ende eine Elektrode 3 mittels ihrer abgestuften Verlängerung 4 eingesetzt ist. Die Verlängerung ist über einen nicht dargestellten Leiter mit dem einen Pol eines HF-Generators verbindbar. Die elektrische Zuleitung kann auch über einen Schaft 5,der auch als flexible Metallwendel ausgebildet sein kann, erfolgen. Der Leiter sowie der Schaft 5 sind zur Vermeidung eines unbeabsichtigten Stromüberganges mit einer äußeren Isolation 6 versehen.

Die sonst kugelförmige Elektrode 3 weist durch eine zu der Längsachse des Sondenschaftes 2 parallel verlaufende Schnittfläche 8 die Form eines Kugelabschnittes 7 auf, so daß dessen Kalottenfläche in eine Richtung quer zu der Längsachse des Sondenschaftes 2 weist. Auf der Abschnittfläche 8 des Kugelabschnittes 7, und zwar in deren bzw. dessen distalem Endbereich, ragt ein unlösbar mit dem Kugelabschnitt 7 verbundener Elektrodenring 9 auf, wobei sich dessen Ringebene im Ausführungsbeispiel senkrecht zu der Längsachse des Sondenschaftes 2 und zu der Abschnittfläche 8 erstreckt. Wie Figur 2 zeigt, ist der Elektrodenring 9 derart geformt, daß er sich mit dem Kugelabschnitt 7 der Elektrode 3 zu einer kreisförmigen Querschnittsfläche ergänzt.

Unabhängig von der in Fig. 2 dargestellten Ringelektrodenraumform können sowohl die Elektrodengeometrie als auch deren Abmessungen frei gewählt werden. Es ist des weiteren auch denkbar, den Winkel zwischen der Abschnittfläche 8 und dem Elektrodenring 9 zu verändern , um dadurch beispielsweise dem Anwender die Handhabung zu erleichtern oder aber um besondere Eingriffe in tiefliegendem Rektum zu ermöglichen.

**Ansprüche**

1. Elektrochirurgisches Instrument mit einem Sondenschaft, an dessen distalem Ende eine mit einer HF-Stromquelle verbindbare Elektrode angeordnet ist, dadurch gekennzeichnet, daß die Elektrode (1) die Form eines Kugelabschnitts(7) aufweist, der mit einem Elektrodenring(9) bestückt ist, der im Winkel zu der ebenen Fläche (8) des Kugelabschnittes (7) aufragend angeordnet ist.

2. Elektrochirurgisches Instrument nach Anspruch 1, dadurch gekennzeichnet, daß sich die Ringebene des Elektrodenringes(9) im wesentlichen senkrecht zu der Längsachse des Sondenschaftes (2) erstreckt.

3. Elektrochirurgisches Instrument nach Anspruch 1 oder 2, dadurch gekennzeichnet, daß der Elektrodenring (9) derart geformt ist, daß die in der Ringebene desselben und durch den Kugelabschnitt (7) verlaufend gedachte Querschnittsfläche eine kreisförmige Kontur aufweist.

4. Elektrochirurgisches Instrument nach einem der Ansprüche 1 bis 3, dadurch gekennzeichnet, daß die Ringebene zu der Längsachse des Sondenschaftes (2) geneigt verläuft.

5. Elektrochirurgisches Instrument nach einem der Ansprüche 1 bis 4, dadurch gekennzeichnet, daß der Elektrodenring(9) mit dem Kugelabschnitt (7) unlösbar verbunden ist.

FIG. 1

FIG. 2

EP 0 397 983 A2